Europäisches Patentamt

(19) European Patent Office

Office européen des brevets

(11) Numéro de publication : **0 181 249**
**B1**

(12) **FASCICULE DE BREVET EUROPÉEN**

(45) Date de publication du fascicule du brevet :
07.06.89

(21) Numéro de dépôt : **85402000.5**

(22) Date de dépôt : **15.10.85**

(51) Int. Cl.⁴ : **D 21 C   9/00**, C 12 P  19/14//
D01F2/00, C08B9/00

(54) Fabrication enzymatique de pâte à usage chimique.

(30) Priorité : 17.10.84 FR 8415886

(43) Date de publication de la demande :
14.05.86 Bulletin 86/20

(45) Mention de la délivrance du brevet :
07.06.89 Bulletin 89/23

(84) Etats contractants désignés :
AT BE CH DE FR GB IT LI NL SE

(56) Documents cités :
DE--C-- 1 045 387
CHEMICAL ABSTRACTS, vol. 84, no. 24, 28 juin 1976,
page 87, abrégé 181829e, Columbus, Ohio, US; & SU -
A - 507 677 (BELORUSSIAN TECHNOLOGICAL INSTI-
TUTE) 25.03.1976
CHEMICAL ABSTRACTS, vol. 94, 1981, page 103,
abrégé 67499d, Columbus, Ohio, US; YU. YU. KATKE-
VICH et al.: "Enzymic hydrolysis of polysaccharides
of wood and straw. Study of the degradation of wood
cellulose by different enzyme complexes" & KHIM.
DREV. 1980, (6), 34-9
CHEMICAL ABSTRACTS, vol. 66, 1967, page 10872,
abrégé 116855z, Columbus, Ohio, US; Y. OGIWARA et
al.: "Hydrolysis of pulp with cellulase" & KAMI-PA
GIKYOSHI 21(4), 209-13(1967)

(73) Titulaire : LA CELLULOSE DU PIN
353, bd du Président Wilson
F-33200 Bordeaux (FR)

(72) Inventeur : Debort, Jean-Pierre
19, rue des Poissonniers
F-33470 Le Teich (FR)
Inventeur : Doignie, Jean-Claude
16bis, Chemin du Capitane
F-33260 La Teste (FR)
Inventeur : Fuentes, Jean-Luc
3, Les Vergers de Couhins
F-33140 Villenave d'Ornon (FR)
Inventeur : Pommier, Jean-Claude
35, Allée de Gascogne
F-33170 Gradignan (FR)

(74) Mandataire : Mulier, René et al
SAINT-GOBAIN RECHERCHE 39, quai Lucien Lefranc
F-93304 Aubervilliers (FR)

## Description

La présente invention a trait à la préparation et à l'utilisation de pâtes à usage chimique obtenues à partir de préparation cellulosiques dérivées du bois.

Le bois est un mélange de divers composants, dont les principaux sont la lignine, les hémi-celluloses et la cellulose. A partir de cette matière première disponible dans la nature, il est possible de fabriquer différents types de pâtes cellulosiques, suivant leur teneur en lignine. La composition de ces pâtes et leur procédé d'obtention déterminent leur application.

Ainsi, on désigne sous l'appellation de pâtes à usages chimiques, ou pâtes à dissoudre, les matières premières pour la fabrication notamment de fibres textiles artificielles, de films et pellicules de cellophane, ou de vernis.

Pour fabriquer du papier, en revanche, les matières premières fibreuses utilisées sont constituées par de la pâte mécanique, qui est simplement du bois broyé, pour l'obtention de papier journal, ou encore une pâte chimique écrue ou blanchie comme celle utilisée pour l'obtention du papier kraft.

On peut, d'une manière générale préparer des pâtes au sulfite ou au sulfate.

Dans le procédé au sulfite, on soumet du bois à une digestion avec une solution de bisulfite d'alcalins ou d'alcalino terreux et d'acide sulfureux et les pâtes obtenues sont destinées à des usages sanitaire, chimique ou papetier.

Dans la perspective de la préparation de pâtes à usage chimique, ce sont essentiellement les procédés au sulfite qui sont mis en œuvre.

Or, les pâtes destinées notamment à la préparation de fibres textiles artificielles, comme par exemple les fibres de viscoses doivent, contrairement aux pâtes à usages sanitaires ou papetiers, présenter des propriétés spécifiques : une très grande pureté chimique en cellulose, des longueurs de chaînes cellulosiques régulières, une importante réactivité.

Pour obtenir de telles propriétés il est nécessaire d'isoler de tous les autres constituants la cellulose contenue dans le bois. Pour y parvenir, on procède usuellement à des traitements chimiques poussés à partir du bois : ces traitements sont difficiles à maîtriser, et générateurs de pollution. On observe notamment une dispersion importante des longueurs de chaînes, ainsi qu'une diminution du rendement par rapport au bois due à la dégradation des chaînes cellulosiques courtes.

En particulier, en vue de l'obtention de fibres de viscose, il convient que la pâte soit caractérisée par une teneur en cellulose élevée, de 90 à 95 %, et un degré de polymérisation moyen (DPm) de l'ordre de 700.

Les mauvaises caractéristiques de la pâte influent considérablement sur les propriétés des fibres préparées à partir de cette pâte : on peut par exemple mentionner l'abaissement des propriétés de résistance mécanique ou une moindre filtrabilité.

Par ailleurs, on ne peut pas, pour ce type d'application, partir d'une pâte papetière, puisqu'elle n'est pas suffisamment purifiée en cellulose. Il est alors difficile d'obtenir une solution filable convenant pour l'obtention de fibres textiles artificielles telles que les fibres de viscose. Une pâte papetière correspond généralement à de la cellulose purifiée par délignification et à des fibres cellulosiques convenablement blanchies, mais qui n'ont pas les caractéristiques de degré de polymérisation moyen (DPm) analogues à celles de pâtes à usage chimique.

L'invention se propose de remédier, au moins partiellement, à ce dernier inconvénient.

On connaît du document DE-C-1 045 387 un procédé de fabrication de viscose à partir d'une pâte à usage chimique obtenue par cuisson acide ou basique d'un matériau cellulosique qui se caractérise en ce qu'on fait agir sur la pâte à usage chimique après raffinage des enzymes destructrices de cellulose afin d'obtenir un degré de polymérisation convenable.

L'invention a pour objet un traitement d'une pâte papetière bisulfitique présentant un degré de polymérisation moyen élevé, de 1 100 environ, en vue de lui donner les caractéristiques d'une pâte à usage chimique.

Suivant l'invention, on fait agir sur la pâte papetière, une préparation enzymatique à base de cellulases fongiques possédant une activité $C_1$ et une activité $C_x$, une activité maximale dans la zone de pH comprise entre 1.5 et 7.5 et à une température inférieure à 80 °C, le milieu réactionnel étant constamment agité, avec un pH compris entre 2 et 7 et une température inférieure à 80 °C pendant l'action de la préparation enzymatique.

L'invention est particulièrement avantageuse puisqu'elle permet de donner à une pâte papetière bisulfitique des caractéristiques de degré de polymérisation moyen analogues à celles d'une pâte à usage chimique, en abaissant le DPm de la cellulose, et donc de préparer une solution filable convenant pour l'obtention de fibres textiles artificielles, telles que les fibres de viscose, sans les traitements dégradants et polluants usuellement nécessaires.

On peut alors fabriquer une solution filable à partir d'une pâte cellulosique ayant subi un traitement chimique moins poussé que les pâtes habituelles pour l'industrie textile. Les pâtes papetières bisulfitiques sont particulièrement avantageuses, car elles comprennent des fibres cellulosiques moins dégradées que celles des pâtes à usage chimique. Ces pâtes sont obtenues avec un rendement beaucoup plus élevé par rapport au bois et sont de ce fait plus économiques.

La préparation enzymatique utilisée dans le cadre de l'invention est à base de cellulases, notamment de cellulases fongiques, qui, de façon connue, sont constituées de divers composants, et certaines possèdent en particulier les activités $C_1$ et $C_x$. L'activité $C_1$ est l'action de la cellobiohydrolase pouvant être dosée sur de la cellulose pure très organisée, telle qu'on la trouve essentiellement dans les fibres de coton. Cette activité se manifeste par la production de cellobiose. L'activité $C_x$ peut être dosée sur de la cellulose modifiée, la carboxyméthylcellulose et elle peut être quantifiée par une chute de la viscosité de la carboxyméthylcellulose ou une augmentation des extrémités réductrices.

Comme dans tous les procédés faisant intervenir des enzymes, un certain nombre de conditions doivent être contrôlées pour atteindre une bonne efficacité du procédé : ainsi la température, le pH, la concentration en enzymes, la durée de leur action peuvent être établis de façon préférée dans la mise en œuvre de l'invention.

Suivant une caractéristique supplémentaire de l'invention, on emploie la préparation enzymatique à une concentration comprise entre 0,05 % et 2 % et de préférence entre 0.4 % et 0.6 %, mesurée par rapport au poids total de pâte cellulosique séchée.

D'autres caractéristiques et avantages de l'invention apparaîtront au cours de la description suivante accompagnée d'exemples de réalisation, en relation avec le tableau 1.

Les exemples réalisés dans le cadre de l'invention sont décrits en s'attachant à un certain nombre de paramètres qui influent plus ou moins sur l'efficacité du procédé. Les paramètres pris en considération sont les suivants : le mode d'agitation, la vitesse d'agitation, la température, le pH, la concentration en solution tampon.

Le mode d'agitation joue de façon connue un rôle prépondérant dans tous les procédés industriels. On a pu observer qu'une agitation caractérisée par une énergie de rotation élevée et la présence de forces de cisaillement importantes favorisait la reproductibilité des résultats. Un malaxeur à double bras est à cet égard avantageusement utilisé.

La vitesse d'agitation doit être suffisante pour permettre la formation du complexe enzyme-substrat, mais elle ne doit toutefois pas être trop importante, car cela risquerait d'abaisser l'efficacité de la préparation enzymatique.

L'activité des préparations enzymatiques est directement liée à la température. C'est pourquoi toutes les étapes du procédé dans lesquelles les enzymes interviennent sont exécutées dans des bains thermostatés. C'est en outre en agissant sur la température, en portant le milieu réactionnel à 100 °C pendant 5 minutes environ que l'on bloque la réaction enzymatique.

L'activité des préparations enzymatiques est également fortement liée au pH. Les enzymes ne supportent pas de brusques variations de pH. On se placera donc dans des conditions de pH convenables.

Un des moyens utilisés pour fixer le pH est la présence d'une solution tampon. On a constaté que la concentration en solution tampon avait un effet sur l'activité enzymatique. On se placera avantageusement à une concentration en solution tampon $CH_3COONa/CH_3COOH$ de 0,01 à 0,2 M, qui convient particulièrement aux enzymes utilisés selon l'invention.

Les exemples 1 à 4 concernent des pâtes papetières traitées selon l'invention.

Le tableau 1 récapitule les valeurs des paramètres intervenant dans le traitement de pâtes papetières pour les exemples 1 à 4 : la nature de la pâte, papetière (P) ; le taux de pâte par rapport au poids total de solution, la nature de la préparation enzymatique, cellulase (C), le taux d'enzyme, le pH et la température de réaction (T °C), la nature de l'agitation (H : malaxeur à hélice), la vitesse d'agitation (tr/min), la durée de la réaction, en heures (h) au moment de la mesure des paramètres, ainsi que le DPm.

## Exemples 1 à 4

### Intervention des enzymes sur une pâte papetière.

Dans ces exemples les enzymes interviennent pour donner à une pâte papetière les caractéristiques d'une pâte à usage chimique. La pâte utilisée est une pâte de fibres longues blanchies préparée de façon connue selon le procédé bisulfite. Elle présente les caractéristiques suivantes : un $DP_m$ élevé, de 1 100 environ, une teneur en alphacellulose de l'ordre de 88 %. Cette pâte contient beaucoup plus d'impuretés, notamment d'hémicelluloses, qu'une pâte à usage chimique.

On fait agir sur cette pâte une préparation enzymatique du type cellulase, la cellulase S 50 000, dérivée du micro-organisme Basidiomycète Sp. Poria qui hydrolyse les celluloses solubles et les carboxyméthylcelluloses (CMC) en polysaccharides et cellobiose. Les caractéristiques de cette préparation sont les suivantes : une activité $C_1$ faible, une activité $C_x$ très forte, un pH d'activité maximum de 4,7 et une température optimale de 50 °C. Les conditions de température et de pression choisies correspondent au maximum d'activité de cette préparation enzymatique.

On a réalisé différents essais avec des concentrations variables d'enzymes exprimées en poids total de pâte : 0,05 %, 0,2 % et 0,75 %. On a établi pour ces différentes concentrations l'évolution du DPm au cours de la réaction. On observe effectivement un abaissement de ce DPm croissant avec la concentration en enzymes ce qui traduit une modification des caractéristiques de la pâte dans le sens souhaité.

EP 0 181 249 B1

A partir de la pâte papetière ainsi traitée, on peut alors mettre en œuvre le procédé viscose.

Tableau 1

| Ex. | Pâte | % | Enz. | % | pH | T °C | Agit. | tr/min | h | DP$_m$ |
|---|---|---|---|---|---|---|---|---|---|---|
| 1 | P | 2 | C | 0,2 | 4 | 40 | H | 500 | 1 | 760 |
| 2 | P | 6 | C | 0,2 | 4 | 40 | H | 500 | 1 | 750 |
| 3 | P | 4 | C | 0,05 | 4 | 40 | H | 500 | 4 | 780 |
| 4 | P | 4 | C | 0,75 | 4 | 40 | H | 500 | 4 | 680 |

## Revendications

1. Procédé de traitement d'une pâte papetière bisulfitique présentant un degré de polymérisation moyen élevé, de 1 100 environ, caractérisé en ce que en vue de lui donner les caractéristiques d'une pâte à usage chimique, on fait agir sur la pâte papetière une préparation enzymatique à base de cellulases fongiques possédant une activité $C_1$ et une activité $C_x$, une activité maximale dans la zone de pH comprise entre 1.5 et 7.5, et à une température inférieure à 80 °C, le milieu réactionnel étant constamment agité, le pH maintenu entre 2 et 7 et la température inférieure à 80 °C pendant l'action de la préparation enzymatique.

2. Procédé selon l'une des revendications précédentes caractérisé en ce que la préparation enzymatique est introduite à une concentration comprise entre 0.05 % et 2 % et de préférence entre 0.4 % et 0.6 % en poids rapporté au poids total de pâte cellulosique séchée.

3. Procédé selon une des revendications 1 ou 2, caractérisé en ce que ladite préparation enzymatique est à base d'une cellulase dérivée du Basidiomycète Sp. Poria.

4. Procédé selon une des revendications 1 à 3, caractérisé en ce que le milieu réactionnel est agité à l'aide d'un agitateur possédant une énergie de rotation élevée et exerçant des forces de cisaillement importantes.

5. Procédé selon la revendication 4, caractérisé en ce que le milieu réactionnel est agité à l'aide d'un malaxeur à double bras.

6. Procédé selon l'une des revendications 1 à 5, caractérisé en ce que le pH est compris entre 4 et 6 et la température entre 40 et 50 °C.

## Claims

1. A method of treating a bisulphitic paper pulp having a high mean degree of polymerization of approximately 1,100, characterized in that, in order to give it the characteristics of a pulp for chemical purposes, there is caused to act upon the paper pulp an enzymatic preparation on a basis of fungic cellulases having a $C_1$ activity and a $C_x$ activity, a maximum activity in the pH range from 1.5 to 7.5, and at a temperature below 80 °C, the reaction medium being continually stirred the pH being maintained between 2 and 7 and the temperature below 80 °C during the action of the enzymatic preparation.

2. A method according to one of the preceding Claims, characterized in that the enzymatic preparation is introduced at a concentration of from 0.05 % to 2 % and preferably of 0.4 % to 0.6 % by weight in relation to the total weight of the dried cellulose pulp.

3. A method according to one of Claims 1 or 2, characterized in that said enzymatic preparation is on a basis of a cellulase derived from Basidiomycete Sp. Poria.

4. A method according to one of Claims 1 to 3, characterized in that the reaction medium is stirred by means of an agitator having a high rotary energy and exerting large shearing forces.

5. A method according to Claim 4, characterized in that the reaction medium is stirred by means of a two-armed mixer.

6. A method according to one of Claims 1 to 5, characterized in that the pH is from 4 to 6 and the temperature from 40 to 50 °C.

4

**Patentansprüche**

1. Verfahren zur Behandlung von Bisulfitpulpe, die einen hohen mittleren Polymerisationsgrad von etwa 1 100 aufweist, dadurch gekennzeichnet, maß man, zur Erzielung der charakteristischen Eigenschaften einer Pulpe für chemische Zwecke, auf die Pulpe eine enzymatische Zubereitung auf der Basis von Pilzcellulasen mit einer Wirksamkeit $C_1$ und einer Wirksamkeit $C_x$ bei einer maximalen Wirksamkeit im pH-Bereich zwischen 1,5 und 7,5 und bei einer Temperatur von weniger als 80 °C einwirken läßt, wobei während der Einwirkung der enzymatischen Zubereitung das Reaktionsmedium ständig gerührt, der pH-Wert zwischen 2 und 7 und die Temperatur auf einem Wert unter 80° gehalten wird.

2. Verfahren nach Anspruch 1, dadurch gekennzeichnet, daß die enzymatische Zubereitung in einer Konzentration zwischen 0,05 und 2 Gew.-%, vorzugsweise zwischen 0,4 und 0,6 Gew.-% in Bezug auf das Gesamtgewicht der getrockneten Zellulose-pulpe zugesetzt wird.

3. Verfahren nach Anspruch 1 oder 2, dadurch gekennzeichnet, daß die enzymatische Zubereitung auf einer von den Basidiomyceten Sp. Poria abgeleiteten Cellulase basiert.

4. Verfahren nach einem der Ansprüche 1 bis 3, dadurch gekennzeichnet, daß das Reaktionsmedium mit Hilfe eines Rührwerkes gerührt wird, das eine hohe Rotationsenergie aufweist und sehr große Scherkräfte aufbringt.

5. Verfahren nach Anspruch 4, dadurch gekennzeichnet, daß das Reaktionsmedium mit Hilfe eines Doppelarmmischers gerührt wird.

6. Verfahren nach einem der Ansprüche 1 bis 5, dadurch gekennzeichnet, daß der pH-Wert zwischen 4 und 6 und die Temperatur zwischen 40 und 50 °C liegt.